# EUROPEAN PATENT APPLICATION

(11) **EP 3 982 371 A1**
(43) Date of publication of application: **13.04.2022**
(21) Application number: 20200934.6
(22) Date of filing: 09.10.2020
(51) Int. Cl.: G16H 10/40, G16H 40/63, G16H 40/67

(54) **REMOTE TESTING**

(71) Applicant: Sportradar AG, 9000 St. Gallen (CH)
(72) Inventor: MARTIN, Anja, 81241 München (DE); MUESER, Dominic, Henderson, NV, 89052 (US)
(74) Representative: Müller-Boré & Partner Patentanwälte PartG mbB

(57) **Abstract**

The present invention relates to a computer-implemented method and system for remote testing a user, in particular anti-doping drug testing an athlete, to be carried out by a client device, in particular a client device of a doping control officer, the method comprising: creating a test case for testing a user; initiating a notification of the user to be tested regarding the created test case; receiving real-time information of taking of a test sample of the user to be tested; wherein at least a part of test case information regarding the test case is stored using blockchain technology.

## Description

The present disclosure relates to a computer-implemented method for remote testing a user, in particular anti-doping drug testing an athlete, a computer program product and devices configured to carry out a computer-implemented method for testing a user.

Taking of a test sample of a person, e.g. for medical analysis or anti-doping drug testing, usually requires the respective person to visit a doctor or other competent professional. Athletes are usually visited by a doping control officer of an anti-doping agency.

However, in cases a user to be tested/patient/athlete is difficult to be visited e.g. due to their location, availability of test personnel or due to quarantine measures, taking of a test sample may be delayed or avoided.

This circumstance becomes increasingly important, in particular in the current COVID-19 crisis, which further complicates taking of test samples of a user to be tested/patient/athlete. Thus, alternative and supplementary measures to collect samples are needed to adequately meet the challenges of the present and future.

One challenge faced is that taking of a test sample is often required to be supervised by a competent and/or authorized person, such as a doctor, a nurse or a doping control officer. On the one hand, this shall ensure correct taking of a test sample so that a meaningful analysis may be carried out. On the other hand, integrity/unsophistication of a test sample taken shall be ensured, in particular in anti-doping drug testing of athletes.

In cases of non- and minimal-invasive test sample taking procedures, a competent person is not necessarily required to be present during taking of such a test sample, so that the user to be tested/patient/athlete may perform the taking of a test sample themselves.

However, it shall be ensured that the test sample is authentic and untampered. Accordingly, there is a need for a way of taking a test sample and supervising the same so that tampering may be avoided or at least impeded.

It is therefore an object of the present invention to provide a fast, flexible and secure solution for collecting a test sample remotely, in particular for collecting of a test sample for anti-doping drug testing of an athlete irrespective of the location of the athlete.

### Summary of the Invention

A first aspect of the present invention relates to a computer-implemented method for remote testing a user, in particular anti-doping drug testing an athlete, to be carried out by a client device, in particular a client device of a doping control officer, the method comprising: creating a test case for testing a user; initiating a notification of the user to be tested regarding the created test case; receiving real-time information of taking of a test sample of the user to be tested; wherein at least a part of test case information regarding the test case is stored using blockchain technology.

A method according to the first aspect provides a digital and secure solution for testing of a user using remote technology, preferably for use with least a physical client device and a physical user device which are in connection, e.g. through a network. A competent person, i.e. a person authorized/trained for ordering and/or conducting a test, in particular a medical/doping test, may contact a user (such as a patient or an athlete), to be tested and may instruct and/or supervise during taking of a test sample specifically while not being physically present. By storing at least a part of information/data of the test case in a database using blockchain technology, data security is enhanced. In particular, integrity and authenticity of the information/data recorded and stored on a digital data storage may be ensured so that manipulation/tampering is avoided or at least impeded.

Advantageously, the method further includes storing of at least a part of the test case information on a data storage and creating one or more unique blockchain hashes for at least a part of the test case information.

In general, blockchain is a linked list of records/blocks that uses hash pointers instead of regular pointers, which enables each block of a blockchain to not only locate the next block but also verify whether the data in that node has been changed. Each block specifically contains a cryptographic hash of the previous block, a timestamp and (transaction) data. Once recorded, the data in any given block of a blockchain cannot be altered without causing alteration of all subsequent blocks and/or failure of the blockchain. Accordingly, a substantially tamper-proof chain of data may be created. A blockchain may be sequentially enlarged, i.e. by adding further blocks, complementing an existing blockchain.

Preferably, at least a part of test case information regarding the test case is stored by a distributed blockchain computer system that includes multiple computing nodes, each computing node storing a copy, or a portion thereof, of the blockchain of the distributed blockchain computer system. Preferably, a centralized blockchain is used, which ensures that only known/identified participants may create a block of a blockchain. Preferably, storing of data in the blockchain is performed by the client device and/or a server device or another device, e.g. a network device in connection with the client device and/or a server device. The above applies also specifically applies for the methods of the second and third aspect of the underlying invention described hereinbelow.

In the underlying application, data stored in one or more blocks of a blockchain may be based on at least a part of a test case information regarding one or more test cases. In particular, the test case information comprises one or more of: personal information of the user to be tested; a test ID of the test case; a time stamp of creation of a test ID; a time stamp of a notification of the user to be tested; a time stamp of receiving real-time information; a location of the user device; a time stamp of an audio recording; a digital signature of at least part of an audio recording; a time stamp of a video recording; a digital signature of at least part of a video recording; a type of a test sample taken; a test kit identification information; a test sample analysis progress and/or a test sample result. Personal information of a user/patient/athlete may be suitable for identifying a user/patient/athlete and may include one or more of: name, address, date of birth, gender, nationality, height, weight, federation, sport, discipline, test pool, etc. Preferably a unique test ID is generated for each test case. Information related to a specific test case may be identified (such as marked or tagged) using said unique test ID so that information may be (specifically bijectively) associated with a test case. A time stamp particularly relates to information identifying when a certain event occurred and is preferably used for logging of a single event and/or a sequence of events. Time stamps regarding a test case may include date and/or time when a test ID is being created, when a notification to a user has been initiated by and/or sent and/or received by the user device of a user/patient/athlete to be tested. Similarly, a time stamp identifying a start of transmittance (sending and/or receiving) of real-time information, such as an audio and/or video recording/stream, may be stored. Also, a location of the user/patient/athlete to be tested may be determined, e.g. using GPS/GLONASS/- mobile communication network, and stored. Furthermore, the type of the test sample obtained from the user, such as e.g. dried blood spot, urine, exhaled breath, saliva, etc., and an identification information of an associated/designated test kit may be stored in the blockchain. In particular, a user/patient/athlete to be tested may have one or more test kits at hand, wherein a specific test kit may be required to be used for a specific test case. Preferably, a test kit has a unique identifier, such as a number or (QR-) code, so that a test kit may be unambiguously associated with a certain user/patient/athlete and/or test case.

By storing at least a part of test case information in one or more blocks of a blockchain, it can be ensured that said test case information are not unknowingly modified/tampered. Consequently, certainty of authenticity and/or integrity of the stored test case information is enhanced. Blocks of the blockchain may be created sequentially in accordance to the occurrence of events, respectively, availability of information. This way, the blockchain relating to a specific test case may be dynamically extended over the progress of a test case. The above also applies for the methods of the second and third aspect of the underlying invention described hereinbelow.

Moreover, a preferred approach for enhancing certainty of validity/integrity/authentication of e.g. an audio/video recording regarding a test case, wherein said recording e.g. documents a taking of a test sample, includes generating one or more digital signatures of the recording and storing the same in one or more blocks of a blockchain. A digital signature of e.g. a video recording may include identification, extraction and/or summarization of characteristic components of a video recording, so that a video may be uniquely identified by its resultant digital signature, similar to a fingerprint. Accordingly, if a video recording stored on a data storage is manipulated, modified and/or replaced, the digital signature of the new video recording does not match with the digital signature of the original video so that an authenticity test of the new video recording will fail. Preferably, a hashed block of a blockchain is generated comprising one or more digital signatures of at least a part of an audio/video recording.

A second aspect of the present invention relates to a computer-implemented method for remote testing a user, in particular anti-doping testing an athlete, to be carried out by a user device of a user to be tested, the method comprising: receiving a notification regarding a test case for testing the user; sending authentication information of the user to be tested; sending real-time information of taking of a test sample of the user to be tested; wherein at least a part of test case information regarding the test case is stored using blockchain technology. Preferably, the user device is a mobile device such as a smartphone, tablet or a laptop.

A method according to the second aspect specifically allows notification and/or supervision of a user (such as a patient or athlete) to be tested regardless of his/her location using a user device, such as a smartphone. In particular, with said method, it is not required that the user/patient/athlete visits a facility for testing or that a competent person (such as a medically trained staff and/or a doping control officer) visits the user/patient/athlete. Thus, a testing may be conducted spontaneously and location independently, wherein communication between the competent person and the user/patient/athlete is established e.g. via a user device and a client device. Also, the taking of a test sample may be supervised and documented, e.g. by a competent person such as the medically trained staff and/or the doping control officer, so that taking of the test sample in compliance with regulations may be certified.

Advantageously, the method further includes storing of at least a part of the test case information on a data storage and creating one or more unique blockchain hashes for at least a part of the test case information. Preferably, at least a part of test case information regarding the test case is stored by a distributed blockchain computer system that includes multiple computing nodes, each computing node storing a copy, or a portion thereof, of the blockchain of the distributed blockchain computer system. Preferably, storing is performed by the user device or a server device or another device, e.g. a network device in connection with the user device and/or a server device.

In particular, test case information may comprise one or more of: personal information of the user to be tested; a test ID of the test case; a time stamp of creation of a test ID; a time stamp of a notification of the user to be tested; a time stamp of receiving real-time information; a location of the user device; a time stamp of an audio recording; a digital signature of at least part of an audio recording; a time stamp of a video recording; a digital signature of at least part of a video recording; a type of a test sample taken; a test kit identification information; a test sample analysis progress and/or a test sample result.

In order to ensure that remote testing is conducted on/with the actual user/patient/athlete to be tested, an authentication of the user/patient/athlete is preferably required. Advantageously, authentication information, preferably of a multi-factor authentication (e.g. two-factor authentication), comprises one or more of: a signature of the user to be tested; a password input into the user device; a finger print; a face id; a photograph; a video recording/stream and/or an audio recording/stream. An exemplary and particularly secure multi-factor authentication may include at least one knowledge factor (e.g. password), at least one possession factor (e.g. specified user device) and/or at least one inherent factor (e.g. a biometric factor).

A third aspect of the underlying invention relates to a computer-implemented method for remote testing a user, in particular anti-doping drug testing an athlete, to be carried out by a server device, the method comprising: generating a test ID of a test case for testing a user; sending a notification regarding a test case to a user device of the user to be tested; receiving and verifying authentication information of the user to be tested; receiving real-time information of taking of a sample of the user to be tested; wherein at least a part of test case information regarding the test case is stored using blockchain technology.

With the method according to a third aspect, a client device of a competent person (such as a person authorized and/or trained to conduct and/or arrange a testing of a user such as a patient or athlete), and a user device of a user (such as a patient or athlete) to be tested may be connected over a network so that remote testing according to aspects of the underlying invention may be conducted. The method may be configured to generate a test ID for a specific test case created by a competent person. Also, the verification of authentication information of the user/patient/athlete to be tested may be performed as well as storing of real-time information, such as e.g. a video recording/stream, for documentation purposes.

Advantageously, the method further includes storing of at least a part of the test case information on a data storage and creating one or more unique blockchain hashes for at least a part of the test case information. Preferably, at least a part of test case information regarding the test case is stored by a distributed blockchain computer system that includes multiple computing nodes, each computing node storing a copy, or a portion thereof, of the blockchain of the distributed blockchain computer system. Storing may be performed by the server device or another device, e.g. a network device in connection with the server device.

Advantageously, test case information comprises one or more of: personal information of the user to be tested; a test ID of the test case; a time stamp of creation of a test ID; a time stamp of a notification of the user to be tested; a time stamp of receiving real-time information; a location of the user device; a time stamp of an audio recording; a digital signature of at least part of an audio recording; a time stamp of a video recording; a digital signature of at least part of a video recording; a type of a test sample taken; a test kit identification information; a test sample analysis progress; a test sample result.

Preferably, the method of any one of the first to third aspect further comprises storing real-time information of taking of a test sample on a data storage, preferably a data storage different to a data storage of the test information stored using blockchain technology, respectively, the distributed blockchain computer system; wherein real-time information comprises one or more of: an audio recording/stream; a video recording/stream; a photograph. Preferably, real-time information regarding taking of a test sample and/or packing of a test sample and/or instruction of the user/patient/athlete to be tested is securely stored on a data storage. Since real-time information may include large data files, it may be advantageous to store one or more digital signatures in a blockchain and store the actual real-time information separately. A corresponding and/or similar advantageous data storage management may reduce costs and/or computational effort. Also, a decentralized data storage approach enhances security of the stored data.

Advantageously, the authentication information, preferably of a multi-factor authentication (e.g. two-factor authentication), comprises one or more of: a signature of the user to be tested; a password input into the user device; a finger print; a face-id; a photograph; a video recording/stream; an audio recording/stream. An exemplary multi-factor authentication may include at least one knowledge factor, at least one possession factor and/or at least one inherent factor (e.g. a biometric factor).

A fourth aspect of the invention relates to a client device for remote testing a user, in particular anti-doping drug testing an athlete, comprising a non-transitory computer-readable storage medium including instructions, wherein executing the instructions cause the client device to carry out the computer-implemented method for remote testing a user in accordance to the first aspect of the underlying invention.

A fifth aspect of the invention relates to a user device of a user to be tested comprising a non-transitory computer-readable storage medium including instructions, wherein executing the instructions cause the client device to carry out the computer-implemented for remote testing a user in accordance to the second aspect of the underlying invention. Preferably, the user device is a mobile device such as a smartphone or a laptop.

A sixth aspect of the invention relates to a server device connectable to a client device for testing of a user, in particular anti-doping drug testing an athlete, and a user device of a user to be tested, in particular of an athlete to be tested, over a network, the server device comprising a non-transitory computer-readable storage medium including instructions, wherein executing the instructions cause the server device to carry out the computer-implemented method for remote testing a user in accordance to the third aspect of the underlying invention. Preferably, the server device comprises and/or has access to information regarding the user to be tested, test protocol(s) and/or one or more storage drives/databases. The server device may also be configured to carry out a method according to the fourth and/or sixth aspect of the underlying invention.

Yet a further aspect of the invention relates to a system for remote testing a user, in particular anti-doping drug testing an athlete, comprising: the client device for remote testing a user according to the fourth aspect of the present invention; the user device of a user to be tested according to the fifth aspect of the present invention; and the server device according to the sixth aspect of the present invention.

In the present invention, a type of a test sample taken preferably comprises one or more of: blood (in particular GEL or EDTA); Dried blood spot (DBS); urine; saliva; exhaled breath; tear fluid; tissue; a swab from an oral cavity, nose or throat and/or other body secretion. These or other samples may be taken from humans and/or animals, e.g. racehorses or show jumpers.

Yet a further aspect of the invention relates to a computer program product, particularly in the form of a signal or data stream or tangibly stored on a computer-readable storage, comprising computer-readable instructions which, when loaded and executed on a suitable system, perform the steps of method for remote testing a user according to the above aspect or a particular embodiment thereof.

The present invention is further explained in detail by the following detailed description and the appended drawings, in which particular embodiments are illustrated by way of example, wherein the present invention is in no way limited by these particular embodiments.

### Brief description of the drawings

- Fig. 1: shows an exemplary process of remote testing a user to be tested;
- Fig. 2: shows an exemplary system for remote testing a user according to an aspect of the invention;
- Fig. 3: shows an architecture diagram of a further exemplary system for remote testing a user;
- Fig. 4: shows an architecture diagram of another exemplary system for remote testing a user.

### Description of particular embodiments

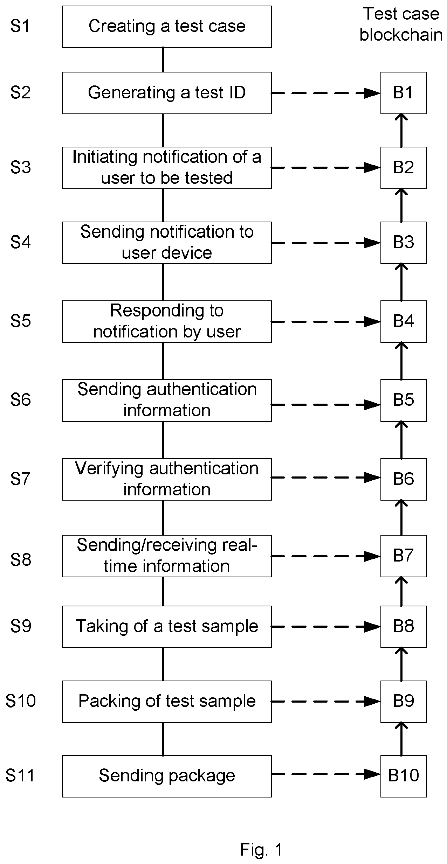

**Fig. 1** shows an exemplary and preferred process of testing a user/patient/athlete to be tested. The present example relates to a particularly preferable process of anti-doping drug testing an athlete, e.g. by a doping control officer of an anti-doping agency. However, a same or similar procedure may be conducted for a common user or a patient to be tested. Accordingly, a competent or authorized person may conduct/supervise the testing. In particular, steps may be omitted, added and/or ordered alternatively.

Preferably, at least a part of test information regarding a test case are recorded/stored on a data storage for logging/documenting the process of the test case. It is thereby particularly advantageous that at least a part of said test information is being securely stored, e.g. by using encryption and/or blockchain technology to avoid/impede manipulation and/or tampering.

Advantageously, a blockchain relating to a specific test case is created comprising a plurality of hashed blocks containing test case information. As illustrated in Fig. 1, a specific event, e.g. S2, causes creation of a hashed block B1 comprising at least a part of test case information relating to S2. A subsequent event, e.g. S3, causes creation of another hashed block B2, which comprises at least a part of test case information relating to S3 and which is linked to B1, thereby building a blockchain for the test case. Accordingly, a test case specific blockchain is created including test case information and, advantageously, a time stamp of creation of one or more of the blocks. A more detailed example is described in the following:
In the following, an example is described with respect to a scenario in which a doping control officer performs or supervises a doping control of an athlete. However, it is to be understood that the present disclosure is equally applicable for performing of a test (e.g. a COVID19-test) of another user (such as a patient) by any competent person such as a (e.g. public) health officer, a medically trained person or the like. In the event that the athlete (as the exemplary user) shall be tested, the doping control officer (as the exemplary competent person) may create a test case (S1) and specify details about the test to be performed. Preferably, the doping control officer may access a database with athlete information, e.g. via a web interface or software tool.

As an example, details about the test to be performed may include one or more of: test date, test time, type of sample taken, etc. Athlete information may include one or more of: name, address, date of birth, gender, nationality, height, weight, federation, sport, discipline, test pool, etc. Optionally, doping control officer may check the stored data and/or add comments and/or modify data.

Based on at least part of said details and/or information, a unique test identification (test ID) is generated (S2). Preferably, said test ID corresponds to a distinctive label for identifying a specific test case and/or its related test case information and/or test sample. By example, the test ID, a time stamp of generating the test ID and preferably further test case information such as e.g. type of sample taken, name, date of birth and gender of the athlete to be tested are stored in a hashed block, which becomes the genesis block of the blockchain of the particular test case. Specifically, the unique test identification (test ID) bijectively identifies the specific test case and/or its related test case information and/or test sample

The doping control officer may schedule a test, i.e. choose a predefined date and/or time for a notification to be sent to a user device, e.g. a smartphone or computer, of the athlete to be tested for notifying the athlete that a test is to be carried out (S3). Alternatively and/or additionally, the doping control officer may initiate notification of the athlete to be tested at any given time. In the present example, a time stamp (specifically identifying the date and/or time that a notification is initiated) is stored in a second hashed block B2, which is linked to the first block/genesis block B1.

Accordingly, a notification is sent to the user device of the athlete to be tested (S4) and, preferably, an associated time stamp is stored in a third hashed block B3, which extends the test case blockchain. Advantageously, a server device connected to the user device and the client device of the doping control officer sends a notification to the user device. The notification may trigger an acoustic and/or optic and/or haptic alarm by/on the user device to notify the athlete.

After having received the notification, the athlete is required to respond to the notification (S5). Such a respond may include opening of a smartphone application or software program on a laptop. Preferably, in case the athlete responds to the notification, a time tamp of the response is stored in a fourth hashed block B4 of the test case blockchain.

Optionally, the athlete may have a specified or predefined time window for responding to the notification. In case the athlete fails to respond to the notification within said specified or predefined time window, the athlete e.g. may be contacted via a separate channel such as via phone, messaging and/or email. Optionally, if unsuccessful, the notification may be deleted and a corresponding note may be recorded/stored. This way, a further attempt of notification of the athlete may advantageously be performed without anticipation by the athlete.

Advantageously, an authentication of the athlete to be tested may be requested. Authentication may include the athlete to send a signature, a password, a finger print, a face id, a photograph, a video recording or stream, an audio recording/stream and/or another kind of response to a challenge (S6). Preferably, a multi-factor authentication is requested, e.g. including a generated code sent to the user for insertion into a corresponding input filed of a smartphone application. Preferably, information regarding the kind of challenge, respectively, the kind of sent authentication information and/or a corresponding time stamp of reception is stored in a further hashed block B5 complementing the test case blockchain.

The authentication is verified (S7), preferably by the server device connected to the user device of the athlete to be tested and/or by the client device of the doping control officer. At least a part of information relating to the authentication process is stored in hashed block B6, in particular an unsuccessful authentication attempt.

It is noted that an authentication procedure (S6 and S7) may take place at a later or earlier stage in the testing process. Accordingly, the corresponding hashed blocks may be positioned at a different location in the test case blockchain.

After successful authentication of the athlete to be tested, real-time information, such as a video recording or stream for example, may be (continuously or intermittently) sent or streamed (particularly in real-time) from the user device to the server device and/or to the client device of the doping control officer (S8).

According to a particular advantageous example, a two-way video feed or stream is established between the client device of the control officer and the user device of the athlete to be tested. This way, a communication during the testing process is enabled between the doping control officer and the athlete.

Preferably, for facilitating documentation of the testing process, at least part of the real-time information is recorded and/or stored on a data storage. Preferably, said information is encrypted and/or hashed in order to avoid tampering/editing of the information stored. Advantageously, one or more time stamps relating to transmittance of real-time information may be stored in a seventh hashed block B7 linked to B6 of the test case blockchain, such as e.g. the start of a video feed/stream.

Optionally, a further person may join the testing process for observation, advantageously via receiving of at least a part of a video feed or stream.

Preferably, the athlete confirms consent to the testing to be carried out. This may include signing of a letter of consent by the athlete and/or confirming his/her consent via the user device. Also, the athlete may receive information e.g. regarding the test to be carried out and/or authorization of the anti-doping agency and/or of the doping control officer. Furthermore, an additional athlete authentication/identification may take place: As an example, athlete information may be presented to the doping control officer specifically via the client device based on which the doping control officer may verify whether the athlete to be tested has actually been contacted and/or has responded. Also, one or more challenges may be created, e.g. a security question to be answered by the athlete. At least a part of corresponding information may be stored or supplemented in one or more hashed blocks of the test case blockchain.

Preferably, the doping control officer provides (via the client device and/or the user device) instructions to the athlete regarding the taking of a test sample. In particular, this may include instructions on how to take the test sample, e.g. location of a smear test, location of a minimal-invasive skin puncture etc. Also, a specific container/test sample kit may be specified to the athlete for taking and/or storing/packing of the taken test sample.

Preferably, taking of a test sample should be carried out according to specified or predefined steps and/or a specified or predefined procedure (S9). The doping control officer and/or the athlete may take notes/comments regarding the taking of the test sample for documentation purposes, which may be stored in a database and/or in the blockchain.

Advantageously, a recording of the taking of a test sample, preferably a video recording, is securely stored on a data storage. For avoiding or at least impeding manipulation, the recording may be encrypted and/or provided with a water mark, e.g. comprising the unique test ID.

Preferably, a hashed block of a blockchain may be generated comprising a hashed digital signature ("fingerprint") of at least part of the video recording. A digital signature specifically is a mathematical scheme for verifying the authenticity of digital messages or documents and that the message/document was not altered. Such a digital signature may be stored in a block B8 of the test case blockchain.

Advantageously, at least part of the blockchain is publicly accessible for providing transparency and verification capability. Alternatively or in addition, a time stamp of taking of the sample and/or a time stamp of a video recording may be stored in a hashed block of the test case blockchain. Preferably, transmittance of real-time information may be terminated after the test sample has been orderly packed/sealed.

The type of sample taken from the user may be for example one or more of: blood (in particular GEL or EDTA); dried blood sample; serum; urine; saliva; exhaled breath; tear fluid; tissue; a swab from an oral cavity, nose or throat; and/or other body secretion. Preferably, a corresponding test kit/packaging should be provided to the athlete. The respective test kit specifically has a unique identifier, such as a number or (QR-) code, so that a test kit may be unambiguously associated with a certain user/patient/athlete and/or test case. The unique identifier of the test kit is stored in a corresponding data base and/or in the blockchain.

Particularly favorable test samples to be taken from a user to be tested are e.g. dried blood spot and/or exhaled breath:
Dried Blood Spot includes a drop of whole blood dried on filter paper and represents a technique for minimally invasive sample collection in a multitude of analytical disciplines, e.g. therapeutic drug monitoring, preclinical drug development and diagnostic analysis of metabolic disorders in newborns. Dried Blood Spot sampling is characterized by cost-effectiveness, straightforwardness, robustness and facilitated storage and shipment conditions. Also, authenticity of a sample - if questioned - may be assured by DNA. Taking a sample for dried blood spot testing may be taken by the user to be tested.

Exhaled breath contains aerosols which originate from lungs and/or blood. These aerosols contain revealing information for diagnostics, therapeutics and analytics. This technique is convenient, reliable and low cost. It is a noninvasive way to collect chemicals in breath e.g. for disease detection, exposure monitoring, and drug metabolism.

After taking of the test sample, the athlete seals and packs the container/test kit (S10). Optionally, a GPS-tracker and/or a logging device may be placed in the package for tracking/monitoring of the package. Advantageously, a logging device may comprise one or more sensors for measuring environmental conditions such as e.g. temperature, air pressure and/or illumination and may store/transmit the data. Preferably the packing and sending process (S10, S11) is supervised by the doping control officer and specifically video recorded or otherwise documented and securely stored on a data storage. In particular, the athlete may upload a photograph of the receipt and/or submit a tracking number of the delivery company. Alternatively, a delivery provider may collect the package. In particular, information to be logged in the system (specifically stored in a database and/or in the blockchain) may include one or more of: agent, date, facility, logistics service provider, waybill or tracking number, driver name/number, driver signature, date & time of the delivery, name of recipient of the package, receiver position and/or receiver signature. At least part of the information regarding sending of the package may be encrypted and/or protected by blockchain technology as outlined above. In particular, information regarding the test kit used and/or a time stamp of packing may be stored in block B9, which is linked to the previously created block B8 of the test case blockchain. Similarly, information regarding sending of a package containing the test sample may be stored in a further block B10 linked to B9.

The further process of analyzing the test sample e.g. in a laboratory is preferably stored on a data storage. Corresponding information may include one or more of: arrival at the laboratory/test center, test status of the test sample, result of the test. Preferably, at least part of this information may be stored in a substantially tamper-proof manner, e.g. by using blockchain technology. Advantageously, upon availability of said information, one or more hashed blocks may be created containing at least part of said information and linked to the existing test case blockchain.

The method shown in Fig. 1 and described above represents a particularly advantageous embodiment of the underlying invention. It should be understood, however, that an order of steps carried in this example may be alternated and/or steps may be omitted and/or combined differently without departing from the present disclosure. Also, additional steps may be carried out, in particular one or more of the following:
- Optionally, the athlete may have carried out an identification process prior of being able to be tested via the present remote testing. As an example, the athlete may have installed an application on a user device such as a smartphone and performed a registration/first time identification process.
- Such a process may include connecting to a server device and authentication e.g. via an invitation code sent by an anti-doping agency.
- The athlete may send authentication information for authentication prior to testing being performed via the remote testing system.
- The athlete may set a way of being contacted, e.g. by an email address and/or a telephone number.
- The athlete may receive one or more test kits for carrying out testing, preferably, beforehand and/or prior to being notified by a doping control officer.
- The doping control officer or the athlete may select a specific test kit available to the athlete for taking of a test sample of the athlete. Preferably, an identifier such as an ID number and/or a QR code provided on a test kit may ensure that the correct test kit is used.
- The test kits sent to the athlete beforehand particularly are pre-registered, i.e. the test kits are provided with the identifier such as the ID number and/or the QR code. Upon selection of the test kit, the athlete retrieves the corresponding identifier (e.g. by scanning the code) of the used kit. Alternatively, the athlete might be required to register a received test kit so that availability of a test kit is apparent to the doping control officer.
- If there occurs the situation that the athlete is not able to attend the testing directly - e.g. because of a medal ceremony - the sending and/or receiving of real-time information may be started and the authorized person/a doping control officer may supervise every movement of the athlete till he/she is ready for testing.
- At least a part of such real-time information may be stored in a database and/or the blockchain for documentation purposes.

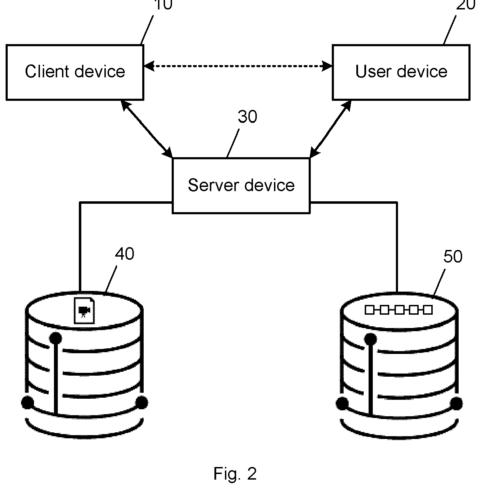

**Fig. 2** shows a an example of a system for remote testing a user including a client device 10, e.g. of an authorized person (such as doping control officer or a health officer) a user device 20 of a user (such as an athlete or patient) to be tested, e.g. a smartphone, and a server device 30 in connection to the client device 10 and/or the user device 20 and, preferably, comprising or having access to one or more databases or data storages 40, 50 for obtaining and/or storing information.

As shown in Fig. 2, the server device 30 may be configured to provide a connection between the client device 10 and the user device 20 e.g. via a network such as the internet or a mobile communication network. Alternatively or in addition, a direct connection between the client device 10 and the user device 20 may be established. Also, one or more further client devices 10 and/or user devices 20 may be provided and/or connected.

In the example of Fig. 2, server device 30 comprises/may access multiple (e.g. two) databases/data storages, namely data storage 40 and data storage 50. This is particularly advantageous because either a backup of at least a part of information to be stored may be created and/or information to be stored may be distributed over a plurality (e.g. 2, 3, 4...) of data storages for increased security and/or advantageous data storage management.

Information to be stored may include test case information such as e.g. personal information of the user to be tested; a test ID; a time stamp of creation of a test ID; a time stamp of a notification of the user to be tested; a time stamp of receiving real-time information; a location of the user device; a time stamp of an audio recording; a digital signature of at least part of an audio recording; a time stamp of a video recording; a digital signature of at least part of a video recording; a type of a test sample taken; a test kit identification information; a test sample analysis progress and/or a test sample result. Advantageously, at least a part of the test case information is stored in a blockchain comprising a plurality of hashed blocks for enhanced security.

Furthermore, information to be stored may include real-time information sent/received by a client device 10 and/or user device 20 and/or server device 30 such as e.g. an audio recording/stream; a video recording/stream and/or a photograph of a taking of a test sample and/or packing of a test sample.

An exemplary client device 10 according to an aspect of the underlying invention is configured to carry out a method for testing a user, wherein, preferably, the client device 10 allows the competent person (such as e.g. the doping control officer), to create a test case for testing the user (such as the athlete). Also, the client device 10 may be configured to initiate a notification to be sent to the user to be tested specifically via the user device 20 in order to inform the user of the test case to be carried out. Preferably, the client device 10 is configured to send and/or receive real-time information to, respectively, from the user to be tested. Such real-time information may include one or more of an audio recording or stream; a video recording/stream and/or a photograph, particularly, for instructing and/or supervising the user to be tested. Advantageously, at least a part of the real-time information and/or at least part of the real-time information is encrypted and/or hashed and stored in a blockchain comprising a plurality of hashed blocks for enhanced security.

An exemplary user device 20 of a user to be tested is configured to carry out (or assist the carrying out of) a method for testing a user, wherein the user device 20 may receive a notification regarding a test case, send authentication information of the user and/or real-time information for documentation of taking of a test sample. Such real-time information may include one or more of an audio recording/stream; a video recording/stream and/or a photograph, in particular for receiving instructions and/or allow supervision of the user to be tested. Preferably, the user device 20 may be a smartphone, tablet or a laptop comprising a camera configured to connect to the client device 10 and/or the server device 30 for sending and/or receiving information.

Advantageously, client device 10, user device 20 and/or server device 30 are configured to (at least temporarily) store information, in particular at least a part of test case information, on one or more data storages, preferably, by creating one or more unique blockchain hashes for at least a part of the test case information.

This is particularly advantageous for test case information for identifying integrity of a test sample/test kit, thereby enhancing protection of the test case information.

As an example, real-time information documenting the taking of a sample may be stored on data storage 40, wherein, preferably, one or more time stamps and/or digital signatures of at least a part of the video is stored on a different data storage 50, preferably using one or more hashed blocks of a blockchain.

As described above, one or more digital signatures of at least a part of real-time information transmitted may be stored in a test case blockchain. By verification of one or more securely stored digital signatures, it can be verified if a recording of real-time information is modified/manipulated. Advantageously, enhanced security may be achieved by storing the real-time information on a data storage separate of the digital signature(s) such as data storage 40 and data storage 50.

Advantageously, at least a part of test case information regarding a test case is stored by a distributed blockchain computer system that includes multiple computing nodes, each computing node storing a copy, or a portion thereof, of the blockchain of the distributed blockchain computer system. This way, an even greater protection of the test case information may be obtained in order to further prevent or at least impede tampering/manipulation. In particular, since each node comprises a copy of at least a portion of the blockchain, all copies would have to be manipulated in order to not corrupt the blockchain.

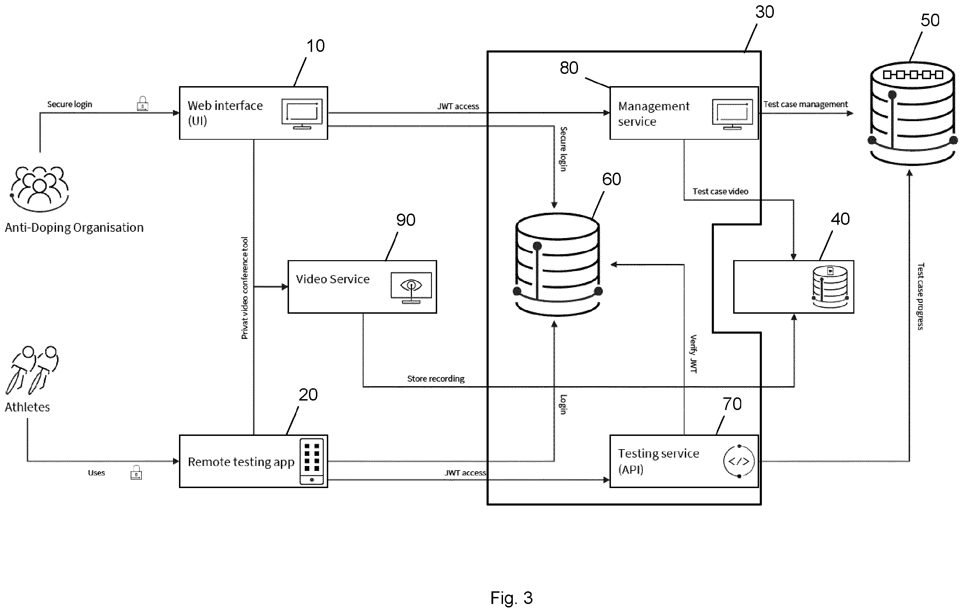

**Fig. 3** shows an architecture diagram of an exemplary system for remote testing a user comprising a client device 10, a user device 20 and a server device 30.

Prior of a testing of a user (such as an athlete) is initiated, the user (athlete) may receive an invitation document, e.g. a welcome letter, from an administrator or authorized person or doping control officer, which ensures the installation of of an application to the user device 20 (such as a user's/athlete's smartphone 20). Specifically, the invitation document includes a QR code for allowing the user to download the specific application to the user device 20.

A registration process may be performed. As an example, the user (athlete) opens the application, inputs (e.g. types) in an invitation access code and/or inputs and/or confirms a phone number.

After registration, the user (athlete) may access the remote testing system and e.g. may input or insert personal information, which will be stored on a data storage 60 of the server device 30. After registration, the user/athlete must be aware that they can be notified at any time for testing, in particular anti-doping drug testing

An authorized person such as a doping control officer of an anti-doping organization or agency may access a web interface on a client device 10. Through the web interface, the doping control officer obtains access (specifically with signature and/or encryption using e.g. a JSON Web Token or JWT access) to management service 80 on a server device 30 for managing test cases. Accordingly, the doping control officer can create a new test case.

Advantageously, the management service 80 has access to a user (athlete) database useful for test case creation. User (athlete) information may be checked and/or modified/adapted by the doping control officer. Using the management service 80, one or more tests may be planned over a time frame, e.g. over a calendar year.

Upon creating and/or planning a test case, a unique test ID is generated for a test case, which is preferably used for tagging and/or identification of information/data relating to the particular test case.

Preferably, test case information is stored on data storage 50 by using blockchain technology. More specifically, a hashed block may be created and stored on at least data storage 50 including at least the unique test ID of the test case.

During the progress of a test case, a corresponding test case blockchain is created and extended including one or more hashed blocks comprising one or more time stamps of specific events and/or comments/notes/information regarding the test case.

At a specified or defined time, the doping control officer may send a notification to the user/athlete to be tested. User (athlete) may accept or decline using a remote testing application on his/her user device 20. If a user (athlete) declines, an unsuccessful attempt may be logged by the management service, preferably in a hashed block of a test case blockchain on data storage 50 (such as a No-SQL (Semi-SQL & Semi-NoSQL)). Append-only database that provides an immutable, transparent and/or cryptographically verifiable transaction log specifically owned by a central authority, e.g. Amazon Quantum Ledger Database (QLDB) provided by Amazon Web Services (AWS)).

The doping control officer can include a comment/note with information, which may be stored in the test case blockchain on data storage 50, together with a time stamp of sending of the notification.

If a user (athlete) accepts, real-time information are transmitted between the client device 10 and the user device 20, e.g. in the form of a video call (video streaming). In the example of Fig. 3, this is performed using a private video conference tool or video service 90 such as Jitsi, MS Teams, Webex, Skype or the like.

Specifically, the doping control officer and the user/athlete are following the procedure for the defined test method for taking of a test sample. A recording of the video(s) documenting the taking of the test sample are stored on data storage 40.

The doping control officer may access the stored video(s) stored on data storage 40 via web interface/management service 80.

Preferably, one or more digital signatures of a video are generated and stored in one or more hashed blocks in the test case blockchain on data storage 50 for ensuring authenticity of the recording(s).

After providing a test sample, the user/athlete packs the test sample together in a provided package. Optionally, a tracking sensor, configured to record environmental data and/or the location/route of the package, may be activated. Advantageously, the package, respectively the test kit, is sealable/tamper-proof. The user/athlete brings the sample to the post office and/or hands it over to a delivery service. Specifically, the user takes a photo of the receipt and/or provides a tracking number and sends it to the server device 30, which stores it in a hashed block of the test case blockchain.

The server device 30 may further comprise or have access to data storage 60 with defined rights of a doping control officer and/or an athlete. In particular, the rights of an athlete may be restricted to accessing only his/her own test cases through testing service using the app installed on his/her user device 20. Accordingly, a user/athlete may access a testing service 70 via the smartphone app for receiving information regarding conducted test cases and may inspect test case information stored on data storage 50. In contrast, a doping control officer may have access to different and/or further information such as e.g. the facility where the test sample is being analyzed.

The further progress of a test case, in particular a test result/report is stored in one or more hashed blocks of the test case blockchain on data storage 50, which doping officer and user/athlete may access through web interface, respectively, remote testing app.

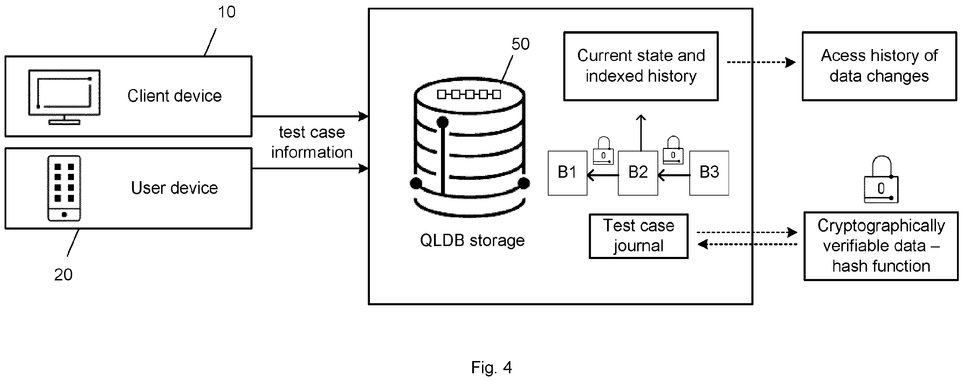

**Fig. 4** shows an architecture diagram of another exemplary system for remote testing a user, wherein the system is preferably based on a ledger database including immutable and transparent, cryptographically verifiable data and comprising a client device 10 and a user device 20.

Test case information (or at least a part thereof) regarding a test case may be transmitted/sent from a client device 10, such as a personal computer or laptop, and/or a user device 20, such as a smartphone, to data storage 50. Data storage 50 may preferably comprise a Quantum ledger database (QLDB). Test case information (or at least a part thereof) may be stored on data storage 50, wherein test case information may in particular include: personal information of the user to be tested, such as e.g. relevant athlete data; a test ID of the test case; a time stamp of creation of a test ID; a time stamp of a notification of the user to be tested; a time stamp of receiving real-time information; a location of the user device; a time stamp of an audio recording; a digital signature of at least part of an audio recording; a time stamp of a video recording; a digital signature of at least part of a video recording; a type of a test sample taken; a test kit identification information; a test sample analysis progress and/or a final test report.

Preferably, a substantially append-only and/or immutable test case journal of changes is generated and stored on data storage 50. The test case journal may include one or more changes of test case information, in particular of a current test case value and/or a test case progress status and/or historical information regarding the test case. A user, in particular a patient and/or an athlete, and/or a competent person, such as a doping control officer, may access the journal to obtain test case information stored in the test case journal. Preferably, a test case journal may include one or more sequent, cryptographically verifiable entries of one or more changes of information, in particular additional and/or updated test case information of the corresponding test case, such as e.g. a test case progress. Advantageously, a single block is created to include at least a part of test case information and/or one or more changes of at least a part of test case information. Blocks are preferably chained together to form a hashed blockchain for increased protection. Preferably, cryptographically verifiable data of at least a part of test case information is obtained by hashing using a hash function, e.g. based on SHA-3, SHA-256, SHA-512, MD5, BLAKE 3 or Keccak-256 standard.

## Claims

1. A computer-implemented method for remote testing a user (1), in particular anti-doping drug testing an athlete, to be carried out by a client device (10), in particular a client device (10) of a doping control officer, the method comprising:
creating a test case for testing a user (S1);
initiating a notification of the user to be tested regarding the created test case (S3);
receiving real-time information of taking of a test sample of the user to be tested (S8);
wherein at least a part of test case information regarding the test case is stored using blockchain technology.

2. Method of claim 1, wherein the test case information comprises one or more of:
personal information of the user to be tested;
a test ID of the test case;
a time stamp of creation of a test ID;
a time stamp of a notification of the user to be tested;
a time stamp of receiving real-time information;
a location of the user device;
a time stamp of an audio recording;
a digital signature of at least part of an audio recording;
a time stamp of a video recording;
a digital signature of at least part of a video recording;
a type of a test sample taken;
a test kit identification information;
a test sample analysis progress;
a test sample result.

3. A computer-implemented method for remote testing a user, in particular anti-doping testing an athlete, to be carried out by a user device (20) of a user to be tested, the method comprising:
receiving a notification regarding a test case for testing the user (S4);
sending authentication information of the user to be tested (S6);
sending real-time information of taking of a test sample of the user to be tested (S8);
wherein at least a part of test case information regarding the test case is stored using blockchain technology.

4. Method of claim 3, wherein the test case information comprises one or more of:
personal information of the user to be tested;
a test ID of the test case;
a time stamp of creation of a test ID;
a time stamp of a notification of the user to be tested;
a time stamp of receiving real-time information;
a location of the user device;
a time stamp of an audio recording;
a digital signature of at least part of an audio recording;
a time stamp of a video recording;
a digital signature of at least part of a video recording;
a type of a test sample taken;
a test kit identification information;
a test sample analysis progress;
a test sample result.

5. Method of claim 3 or 4, wherein the authentication information, preferably of a two-factor authentication, comprises one or more of:
a signature of the user to be tested;
a password input into the user device;
a finger print;
a face-id;
a photograph;
a video recording/stream;
an audio recording/stream.

6. A computer-implemented method for remote testing a user, in particular anti-doping drug testing an athlete, to be carried out by a server device (30), the method comprising:
generating a test ID of a test case for testing a user (S2);
sending a notification regarding a test case to a user device (20) of the user to be tested (S4);
receiving and verifying authentication information of the user to be tested (S7);
receiving real-time information of taking of a test sample of the user to be tested (S8);
wherein at least a part of test case information regarding the test case is stored using blockchain technology.

7. Method of claim 6, wherein the test case information comprises one or more of:
personal information of the user to be tested;
a test ID;
a time stamp of creation of a test ID;
a time stamp of a notification of the user to be tested;
a time stamp of receiving real-time information;
a location of the user device;
a time stamp of an audio recording;
a digital signature of at least part of an audio recording;
a time stamp of a video recording;
a digital signature of at least part of a video recording;
a type of a test sample taken;
a test kit identification information;
a test sample analysis progress;
a test sample result.

8. Method of claim 6 or 7, the method further comprising:
storing real-time information of taking of a test sample on a database;
wherein real-time information comprises one or more of:
an audio recording/stream;
a video recording/stream;
a photograph.

9. Method of any one of claims 6 to 8, wherein the authentication information, preferably of a two-factor authentication, comprises one or more of:
a signature of the user to be tested;
a password input into the user device;
a finger print;
a face-id;
a photograph;
a video recording/stream;
an audio recording/stream.

10. A client device (10) for remote testing a user, in particular anti-doping drug testing an athlete, comprising a non-transitory computer-readable storage medium including instructions, wherein executing the instructions cause the client device (10) to carry out the computer-implemented method of claim 1 or 2.

11. A user device (20) of a user to be tested, in particular of an athlete to be tested, comprising a non-transitory computer-readable storage medium including instructions, wherein executing the instructions cause the client device (20) to carry out the computer-implemented method of any one of claims 3 to 5.

12. A server device (30) connectable to a client device (10) for remote testing a user, in particular anti-doping drug testing an athlete, and a user device (20) of a user to be tested over a network, the server device (30) comprising a non-transitory computer-readable storage medium including instructions, wherein executing the instructions cause the server device (30) to carry out the computer-implemented method of any one of claims 6 to 10.

13. A system for remote testing a user, in particular anti-doping drug testing an athlete, comprising:
the client device (10) for remote testing a user, in particular anti-doping drug testing an athlete, of claim 10;
the user device (20) of a user to be tested, in particular of an athlete to be tested, of claim 11; and
the server device (30) of claim 12.

14. A computer program product comprising computer-readable instructions which, when loaded and executed on a suitable system, perform the steps of method for remote testing a user (1) according to any one of the preceding claims 1 to 9.
